# DEMANDE DE BREVET EUROPEEN

(11) **EP 0 788 745 A1**
(43) Date de publication de la demande: **13.08.1997**
(21) Numéro de dépôt: 96200309.1
(22) Date de dépôt: 09.02.1996
(51) Int. Cl.: A23J 3/12, A23J 1/04, A23J 1/06

(54) **Agents d'inhibition de la croissance des cristaux de la glace**

(71) Demandeur: SOCIETE DES PRODUITS NESTLE S.A., 1800 Vevey (CH)
(72) Inventeur: Jann, Alfred, F-74200 Marin-Thonon (FR); Lundheim, Rolv, N-7010 Trondheim (NO)
(74) Mandataire: Wavre, Claude-Alain

(57) **Abrégé**

Agents d'inhibition de la croissance des cristaux de la glace et procédé de préparation d'un extrait de ces agents dans lequel, on extrait le sang de *Zoarces vivparus*, on le refroidit, on recueille le surnageant constituant le sérum de *Zoarces viviparus* contenant les agents d'inhibition de la croissance des cristaux de la glace, puis l'on congèle le surnageant.
La présente invention concerne également un procédé d'utilisation de ces agents pour la fabrication d'un produit alimentaire.

## Description

La présente invention a pour objet des agents d'inhibition de la croissance des cristaux de la glace, un procédé de préparation de tels agents et un procédé d'utilisation de ces agents dans la fabrication d'un produit alimentaire.

Il est connu que des agents d'inhibition de la croissance des cristaux de la glace dénommés "protéines de l'hystérésis thermique" ont la capacité de se fixer sur les cristaux de la glace et de diminuer leur croissance (Biophysical Journal, February 1991, p 409 - 418). Deux propriétés de ces agents ont été mises en évidence: ils ont la capacité de diminuer la température de congélation apparente d'une solution sans en affecter sa température de décongélation et ils ont également la capacité d'inhiber la recristallisation des cristaux de la glace (Cryobiology, vol. 25, 1988, p 55 - 60).
De plus, trois types d'agents d'inhibition de la croissance des cristaux de la glace ont été mis en évidence chez certains poissons de l'Arctique et de l'Antarctique notamment (The FASEB Journal, May 1990, p 2460 - 2468). Ces agents sont de structure protéique ou de structure glycoprotéique. Ils ont été isolés du plasma ou du sérum de ces poissons puis purifiés. Mais, ils sont également présents dans d'autres tissus.

Il est également connu d'utiliser des agents d'inhibition de la croissance des cristaux de la glace pour améliorer la qualité de produits alimentaires, tels que les desserts congelés, les pâtes congelées ou les produits frais, comme les tomates. DNA Plant Technology Corp a synthétisé une protéine artificielle qui est un agent d'inhibition de la croissance des cristaux de la glace, semblable à ceux que l'on peut trouver chez certains poissons ou d'autres organismes pouvant vivre dans des conditions de températures très basses (Food Processing, October 1992, p 55).

Mais, à ce jour, aucun agent d'inhibition de la croissance des cristaux de la glace n'a été isolé chez *Zoarces viviparus*, poisson vivant notamment sur les côtes de la Norvège et sur les côtes de la mer baltique.

La présente invention a pour but de proposer de nouveaux agents d'inhibition de la croissance des cristaux de la glace, présentant un niveau d'activité remarquable et peuvant être avantageusement utilisés notamment dans la congélation ou la texturation de produits, tels que les produits alimentaires, par exemple.

A cet effet, les agents d'inhibition de la croissance des cristaux de la glace selon la présente invention sont des agents extraits de *Zoarces viviparus*. Ces agents peuvent être de nature protéique, par exemple.

On a constaté avec surprise que ces agents d'inhibition de la croissance des cristaux de la glace permettent effectivement de diminuer la taille des cristaux de glace, lors de la congélation de produits alimentaires, tels que les glaces, les desserts congelés ou les pâtes congelées, par exemple, et confèrent ainsi à ces derniers une texture plus onctueuse.

Dans la suite de la description, on emploiera l'expression "agent d'inhibition des cristaux" dans le sens d'"agent d'inhibition de la croissance des cristaux de la glace".

Dans la suite de la description, on emploiera l'expression "taille typique des cristaux" dans le sens de "taille des cristaux de la glace retrouvée majoritairement".

Dans la suite de la description, on emploiera l'expression "diamètre équivalent" dans le sens de "diamètre d'un cercle qui a la même surface que l'image du cristal considéré".

Dans le procédé de préparation d'un extrait d'agents d'inhibition de la croissance des cristaux de la glace selon la présente invention, on prépare du sérum de *Zoarces viviparus* contenant lesdits agents.

Pour ce faire, on peut extraire le sang de *Zoarces viviparus*, on peut le refroidir, on peut recueillir le surnageant constituant le sérum de *Zoarces viviparus* contenant les agents d'inhibition de la croissance des cristaux de la glace, puis on peut congeler le surnageant, par exemple.

On peut extraire le sang de *Zoarces viviparus* à l'aide d'un capillaire traité, notamment un capillaire traité à l'héparine de sodium, par exemple.

On peut refroidir le sang de *Zoarces viviparus* à 0-5° C, de manière, notamment, à inhiber l'activité des protéases et des peptidases contenues dans le sang, par exemple.

On peut recueillir le surnageant constituant le sérum de *Zoarces viviparus* contenant les agents d'inhibition de la croissance des cristaux de glace, en centrifugeant le sang de *Zoarces viviparus* à 1000-5000 g à 0-5° C pendant 5-15 min, par exemple. Pour ce faire, on peut utiliser une centrifugeuse du type Heraeus Minifuge GL, commercialisée par Heraeus SA, 23, route des Jeunes, CH - 1227 Carouge-Genève par exemple.

On peut congeler le surnageant à une température comprise entre -15° C et - 40° C, par exemple.

La présente invention concerne également un procédé d'utilisation des agents d'inhibition de la croissance des cristaux de la glace extraits de *Zoarces viviparus* dans la fabrication d'un produit alimentaire, dans lequel on incorpore 0,01-10% de sérum de *Zoarces viviparus* à un produit alimentaire au cours de sa préparation.

Les agents d'inhibition de la croissance des cristaux selon la présente invention sont décrits plus en détails par l'intermédiaire des différentes propriétés déterminées notamment à l'aide de différents tests ci-après. Les pourcentages sont donnés en poids, sauf indication contraire.

### Mise en évidence de la cristallisation des cristaux de la glace dans une solution de saccharose à 20% en présence de sérum de Zoarces viviparus:

On prépare 6 échantillons de 1 ml d'une solution de saccharose à 20%, auxquels on ajoute, à différentes concentrations, du sérum de *Zoarces viviparus*, contenant les agents d'inhibition des cristaux.

Les échantillons sont gardés au réfrigérateur, avant d'être placés sous un microscope de type Polyvar, commercialisé par Reichert-Jung, Harnalser Hauptstrasse 219, AT - 1170 Wien et d'être refroidis rapidement jusqu'à une température de - 100° C à l'aide d'un contrôleur de températures de type Lincam, commercialisé par Lincam Scientific Instruments LTD, Epsom Downs Metro Centre, Waterfield, Tadworth, Surrey, KT205HT - UK.

Puis l'on réchauffe les échantillons jusqu'à une température de -9° C et l'on observe, au microscope de type Polyvar, la taille des cristaux contenus dans les échantillons ainsi préparés dans un intervalle de temps de 30 à 120 min.

Parallèlement, on prépare, dans les mêmes conditions, un échantillon témoin contenant 1 ml d'une solution de saccharose à 20%.

Les % de sérum de *Zoarces viviparus* contenant les agents d'inhibition des cristaux que l'on ajoute aux échantillons d'une solution de saccharose à 20%, sont donnés en volume.

Echantillon 1: A 1 ml d'une solution de saccharose à 20%, on ajoute 1,3% de sérum de *Zoarces viviparus* puis l'on prépare l'échantillon, comme indiqué ci-dessus. Après 60 min à -9° C, les cristaux ont une taille typique inférieure à 2 µm. On n'observe aucune évolution de la taille des cristaux au cours du temps.

Echantillon 2: A 1 ml d'une solution de saccharose à 20%, on ajoute 1% de sérum de *Zoarces viviparus* puis l'on prépare l'échantillon, conune indiqué ci-dessus. Après 30 min à -9° C, les cristaux ont une taille typique inférieure à 2 µm. On n'observe aucune évolution de la taille des cristaux au cours du temps.

Echantillon 3: A 1 ml d'une solution de saccharose à 20%, on ajoute 0,1% de sérum de *Zoarces viviparus* puis l'on prépare l'échantillon, comme indiqué ci-dessus. Après 30 min à -9° C, les cristaux ont une taille typique inférieure à 15 µm. La taille des cristaux évolue très peu au cours du temps. Après 120 min à -9° C, la taille typique des cristaux reste inférieure à 15 µm et les cristaux présentent des formes avec des angles prononcés.

Echantillon 4: A 1 ml d'une solution de saccharose à 20%, on ajoute 0,02% de sérum de *Zoarces viviparus* puis l'on prépare l'échantillon, comme indiqué ci-dessus. La taille des cristaux évolue légèrement entre 60 min et 90 min à -9°C. La taille typique des cristaux est inférieure à 20 µm après 90 min à -9° C. Les cristaux présentent des formes avec des angles prononcés.

Echantillon 5: A 1 ml d'une solution de saccharose à 20%, on ajoute 0,013% de sérum de *Zoarces viviparus* puis l'on prépare l'échantillon, comme indiqué ci-dessus. On observe la même évolution de la taille des cristaux que pour l'échantillon préparé avec 0,02% de sérum de *Zoarces viviparus*. La taille typique des cristaux est supérieure à 20 µm après 90 min à -9° C. Les cristaux présentent des formes avec des angles légèrement arrondis.

Echantillon 6: A 1 ml d'une solution de saccharose à 20%, on ajoute 0,01% de sérum de *Zoarces viviparus* puis l'on prépare l'échantillon, comme indiqué ci-dessus. On observe une évolution de la taille des cristaux plus importante que dans l'échantillon préparé avec 0,013% de serum de *Zoarces viviparus*. De plus, les cristaux ont des formes plus arrondies que dans l'échantillon préparé avec 0,013% de sérum de *Zoarces viviparus*. La taille typique des cristaux est supérieure à 25 µm, après 90 min à -9° C

Echantillon témoin: On prépare une solution de saccharose à 20% comme indiqué ci-dessus mais sans y ajouter du sérum de *Zoarces viviparus*. Les cristaux grandissent vite. Leur forme est arrondie et après 90 min à -9° C, la taille typique des cristaux est supérieure à 25 µm.

Ainsi, si l'on ajoute à une solution de saccharose à 20% du sérum de *Zoarces viviparus* à une concentration de 1,3 à 0,013%, on observe au microscope des cristaux de glace relativement petits et dont la taille n'évolue que très peu au cours du temps.

Par contre, si l'on ajoute à une solution de saccharose à 20% une très faible concentration de sérum de *Zoarces viviparus*, les cristaux de glace grandissent plus rapidement.

Entre l'échantillon 6 et l'échantillon témoin, on est à la limite où l'on observe très peu de différences dans la forme et dans l'évolution de la taille des cristaux de glace.

Si la concentration de sérum de Zoarces viviparus contenu dans une solution de saccharose à 20% est comprise entre 0,01% et 10%, on constate que les formes des cristaux présentent des angles plus prononcés que celles des cristaux d'une solution témoin ne contenant pas ledit sérum.

### Mesure de l'hystérésis thermique:

Cette analyse est effectuée à l'aide d'un contrôleur de températures de type Clifton Nanolitre Osmomètre, commercialisé par Clifton Technical Physics, P.O. Box, Hartford, US - New York, 12830 et d'un stéréomicroscope de type Wild MZ8, commercialisé par Leica A.G, Verkaufsgesellschaft, Kanalstrasse 21, CH - 8152 Glattbrugg.

Pour ce faire, on prélève une fraction de 20 nl d'un échantillon de sérum de *Zoarces viviparus* à température ambiante et on la dépose dans un réceptacle placé dans le contrôleur de températures. Ledit réceptacle contient de la paraffine liquide. On effectue une congélation rapide suivie d'une décongélation progressive, on observe à l'aide d'un stéréomicroscope la décongélation des cristaux. Puis l'on ralenti et l'on arrête la décongélation, de manière à stabiliser le seul cristal de petite taille restant et l'on note alors la température. Cette température est le point de décongélation.
Puis, l'on refroidit doucement et l'on note la température à laquelle le cristal commence à grossir. Cette température est le point de congélation apparente.

On calcule alors la valeur de l'hystérésis thermique en calculant la différence entre le point de congélation apparente et le point de décongélation. La valeur de l'hystérésis thermique de la fraction de sérum de *Zoarces viviparus* est de 1,4° C, ce qui correspond à une valeur élevée pour un poisson contenant de tels agents.

### Etude comparative de l'activité d'agents d'inhibition de la formation des cristaux contenus dans un sérum de poisson:

On mesure l'hystérésis thermique d'échantillons de sérum extrait de *Gadus morhua*, poisson vivant sur les côtes de Terre-Neuve, *Pollachius pollachius*, poisson vivant sur les côtes de la Méditerranée jusqu'au nord de la Norvège, *Gobiuscullus flavescens*, poisson vivant sur les côtes européennes et sur les côtes japonnaises, *Myoxocephalus scorpius*, poisson vivant dans l'Arctique et près des côtes de la Norvège, *Liparis liparis*, poisson vivant sur les côtes de l'Atlantique et *Spinachia spinachia*, poisson vivant dans la baie de Biscay et jusqu'au nord de la Norvège. On compare les valeurs de l'hystérésis thermique de ces différents échantillons avec la valeur de l'hystérésis thermique d'un échantillon de sérum de *Zoarces viviparus*.

Tous les poissons ont été capturés en hivers dans un fjord norvégien et ont été tués au maximum 3 h avant d'effectuer les mesures de l'hystérésis thermique à partir d'échantillons de sérum extraits de ces poissons.

Pour ce faire, on procède de la manière décrite dans le test "Mesure de l'hystérésis thermique".

Les valeurs de l'hystérésis thermique de ces échantillons de sérum sont mentionnées dans le tableau I ci-après:

**Tableau I**

| **échantillon de sérum extrait de** | **valeur de l'hystérésis thermique (° C)** |
|---|---|
| *Gadus morhua* | 0 |
| *Pollachius pollachius* | 0 |
| *Gobiuscullus flavescens* | 0,4 |
| *Myoxocephalus scorpius* | 0,1 |
| *Spinachia spinachia* | 0,2 |
| *Liparis liparis* | 0,4 |
| *Zoarces viviparus* | 1,4 |

Les valeurs de l'hystérésis thermique ainsi mesurées permettent de mettre en évidence le fait que les agents d'inhibition des cristaux contenus dans le sérum de *Zoarces viviparus* présentent une activité supérieure à celles des agents d'inhibition des cristaux contenus dans le sérum d'autres poissons vivant dans des conditions similaires.

### Mesure de la taille des cristaux de la glace dans la crème glacée contenant du sérum de Zoarces viviparus:

On mesure la taille des cristaux de la glace dans une crème glacée contenant du sérum de *Zoarces viviparus*, puis l'on compare ces résultats avec la taille de cristaux de la glace dans une crème glacée produite dans des conditions identiques, mais ne contenant pas de sérum de *Zoarces viviparus*.

Pour ce faire, on utilise une crème glacée contenant 0,05% de sérum de *Zoarces viviparus* que l'on a stocké à -40° C. On scie des portions de 1cm³ au milieu de la masse de la crème glacée puis on les transfère dans une enceinte réfrigérée à -10° C.

Dans un tube de caoutchouc silicone on prépare, à -10° C, un échantillon contenant 3 mm³ de graisse silicone, 3 mm³ d'une desdites portions et 7 gouttes de benzine de pétrole. On ferme le tube aux deux extrémités et l'on mélange son contenu à l'aide d'une tige en verre.

On transfère l'échantillon sur une lame porte-objet de microscope et on le recouvre d'une seconde lame porte-objet de microscope.

On place l'échantillon ainsi préparé sous le microscope optique et l'on visualise son image sur écran vidéo. A l'aide du programme *PC-IMAGE* commercialisé par Gloor Instrumente AG, Brauereistrasse 10, CH - 8610 Uster, on obtient une image binaire représentative des cristaux. A l'aide dudit programme, on mesure la taille des cristaux exprimée en diamètre équivalent.

On mesure la taille des cristaux de la glace dans un témoin que l'on prépare comme décrit ci-dessus à partir d'une crème glacée ne contenant pas de sérum de *Zoarces viviparus*.

Puis l'on compare, selon le critère de la taille, la distribution des cristaux de la glace dans la crème glacée contenant du sérum de *Zoarces viviparus* par rapport au témoin.

La distribution des cristaux de la glace dans la crème glacée contenant du sérum de *Zoarces viviparus* (a) et celle des cristaux de la glace dans le témoin (b), selon le critère de la taille, est indiquée dans le tableau II ci-après:

**Tableau II**

| taille des cristaux (µm) | distribution dans (a) | distribution dans (b) |
|---|---|---|
| 0-10 | 1 | 0 |
| 10-20 | 162 | 50 |
| 20-30 | 192 | 132 |
| 30-40 | 116 | 128 |
| 40-50 | 50 | 76 |
| 50-60 | 14 | 27 |
| 60-70 | 12 | 12 |
| 70-80 | 0 | 4 |
| 80-90 | 3 | 0 |
| 90-100 | 0 | 0 |
| 100-110 | 0 | 0 |

On constate, à partir des résultats mentionnés dans le tableau II, que les cristaux de petite taille sont présents en plus grand nombre dans la crème glacée contenant du sérum de *Zoarces viviparus* (a). En effet, on peut mettre en évidence le fait que, dans la crème glacée contenant du sérum de *Zoarces viviparus* (a), les cristaux sont distribués majoritairement dans une fourchette de tailles allant de 10 à 40 µm, alors que, dans le témoin (b), les cristaux sont distribués majoritairement dans une fourchette de tailles allant de 20 à 50 µm.

De plus, à partir des valeurs de taille de l'ensemble des cristaux, on calcule la taille moyenne des cristaux dans la crème glacée contenant du sérum de *Zoarces viviparus* et l'on compare cette valeur à la taille moyenne des cristaux de la glace dans le témoin. La taille moyenne des cristaux de la glace dans la crème glacée contenant du sérum de *Zoarces viviparus* est de 27,8 µm et celle des cristaux de la glace dans le témoin est de 33,8 µm.

### Mesure de la taille des cristaux de la glace dans la crème glacée contenant du sérum de Zoarces viviparus après un choc thermique:

On soumet la crème glacée contenant du sérum de *Zoarces viviparus* et le témoin à un choc thermique pendant 36 h. Le choc thermique consiste en un cycle de températures, au cours duquel on réchauffe à -4° C puis l'on refroidit à -20° C et enfin on réchauffe et l'on refroidit dans l'intervalle de températures compris entre -4° C et -20° C.

On procède alors de la manière décrite dans le test ci-dessus.

On compare, selon le critère de la taille, la distribution des cristaux de la glace dans la crème glacée contenant du sérum de *Zoarces viviparus* par rapport à celle des cristaux de la glace dans le témoin.

La distribution des cristaux de la glace dans la crème glacée contenant du sérum de *Zoarces viviparus* (c) et celle des cristaux de la glace dans le témoin (d), selon le critère de la taille, est indiquée dans le tableau III ci-après.

**Tableau III**

| taille des cristaux (µm) | distribution dans (c) | distribution dans (d) |
|---|---|---|
| 0-10 | 0 | 0 |
| 10-20 | 1 | 1 |
| 20-30 | 37 | 0 |
| 30-40 | 61 | 2 |
| 40-50 | 61 | 8 |
| 50-60 | 40 | 20 |
| 60-70 | 18 | 25 |
| 70-80 | 14 | 40 |
| 80-90 | 10 | 37 |
| 90-100 | 3 | 41 |
| 100-110 | 6 | 30 |
| 110-120 | 6 | 18 |
| 120-130 | 4 | 18 |
| 130-140 | 1 | 9 |
| 140-150 | 0 | 8 |
| 150-160 | 0 | 6 |
| 160-170 | 0 | 8 |
| 170-180 | 0 | 1 |
| 180-190 | 0 | 3 |
| 190-200 | 0 | 3 |

Les résultats mentiounés dans le tableau III mettent en évidence le fait que, après un choc thermique, les cristaux de la glace dans une crème glacée à laquelle on ajoute du sérum de *Zoarces viviparus* contenant les agents d'inhibition de la croissance des cristaux sont de taille inférieure à celle des cristaux de la glace de la crème glacée à laquelle on n'a pas ajouté ledit sérum.

En effet, en présence de sérum de *Zoarces viviparus*, 76% des cristaux de la glace dans une crème glacée sont de taille inférieur à 60 µm. Alors que dans le témoin, seulement 11% des cristaux de la glace sont de taille inférieure à 60 µm.

De plus, en présence de sérum de *Zoarces viviparus*, seulement 6% des cristaux de la glace dans une crème glacée sont de taille supérieure à 100 µm. Alors que dans le témoin, 38% des cristaux sont de taille supérieure à 100 µm.

Enfin, à partir des valeurs de taille de l'ensemble des cristaux on calcule la taille moyenne des cristaux de la glace dans la crème glacée contenant du sérum de *Zoarces viviparus* et ayant subi un choc thermique. Puis, l'on compare cette valeur à la taille moyenne des cristaux de la glace dans le témoin. La taille moyenne des cristaux de la glace dans la crème glacée contenant du sérum de *Zoarces viviparus* est de 50,8 µm et celle des cristaux de la glace dans le témoin est de 96,5 µm.

On met donc en évidence le fait que la recristallisation des cristaux de la glace est partiellement inhibée dans une crème glacée à laquelle on a ajouté du sérum de *Zoarces viviparus* contenant les agents d'inhibition de la croissance des cristaux et à laquelle on a fait subir ensuite un choc thermique.

L'exemple suivants est présentés à titre d'illustration d'une utilisation industrielle, dans le domaine alimentaire, des agents d'inhibition de la croissance des cristaux selon la présente invention. Dans l'exemple ci-après, les pourcentages sont donnés en poids, sauf indication contraire.

### Exemple 1

On utilise du sérum de *Zoarces viviparus* contenant les agents d'inhibition de la croissance des cristaux pour la fabrication d'une crème glacée.

Pour ce faire, on dissout dans 494 g d'eau à 65° C, 92,3 g de lait écrémé en poudre, 150 g de saccharose, 26,2 g de sirop de glucose et 5 g d'émulsifiant.

On y ajoute 4 g d'arôme de vanille et 228,5 g de crème à 35% de matière grasse.

On homogénéise cette préparation dans un homogénéisateur de type Rannie, commercialisé par Kindler Maschinen AG, Postfach 297, CH-8021 Zürich, en deux passages successifs, le premier à 140 bar et le second à 40 bar.

On pasteurise la préparation homogénéisée à 83° C pendant 30 s dans un échangeur à plaques.

On la refroidit à 4° C et on la laisse reposer 12 h à cette température, avant d'ajouter 0,05% de sérum de *Zoarces viviparus* et d'effectuer le glaçage dans un freezer de type HOYER MF50 commercialisé par APV TECHNOHOY, Axel Kiers Vej 28-30, DK-8270 Aarhus-Hojbjerg

On obtient ainsi une crème glacée présentant une texture mousseuse.

On durcit ensuite cette crème glacée dans une cellule de refroidissement à air pulsé et on la stocke à -35° C.

Après tempérage à -18° C, cette crème glacée présente une texture lisse et onctueuse.

## Revendications

1. Agents d'inhibition de la croissance des cristaux de la glace extraits de *Zoarces viviparus*.

2. Agents d'inhibition de la croissance des cristaux de la glace selon la revendication 1, caractérisés en ce qu'ils sont de nature protéique.

3. Procédé de préparation d'un extrait d'agents d'inhibition de la croissance des cristaux de la glace, dans lequel on prépare du sérum de *Zoarces viviparus* contenant lesdits agents.

4. Procédé selon la revendication 3, dans lequel:
- on extrait le sang de *Zoarces vivparus*,
- on le refroidit,
- on recueille le surnageaut constituant le sérum de *Zoarces viviparus* contenant les agents d'inhibition de la croissance des cristaux de la glace,
- puis l'on congèle le surnageant.

5. Procédé selon la revendication 4, dans lequel on extrait le sang de *Zoarces viviparus* à l'aide d'un capillaire traité.

6. Procédé selon la revendication 4, dans lequel on refroidit le sang à 0-5° C.

7. Procédé selon la revendication 4, dans lequel on recueille le surnageant constituant le sérum de *Zoarces viviparus* contenant les agents d'inhibition de la croissance des cristaux de la glace, en centrifugeant le sang de *Zoarces viviparus* à 1000-5000 g à 0-5° C pendant 5-15 min.

8. Procédé selon la revendication 4, dans lequel on congèle le surnageant à une température comprise entre -15° C et - 40° C.

9. Procédé d'utilisation d'agents d'inhibition de la croissance des cristaux de la glace extrait de *Zoarces viviparus* pour la fabrication d'un produit alimentaire, dans lequel on incorpore 0,01-10% de sérum de *Zoarces viviparus* à un produit alimentaire, au cours de sa préparation.
